Europäisches Patentamt

European Patent Office

Office européen des brevets

(19)

(11) Numéro de publication : **0 270 643 B1**

# FASCICULE DE BREVET EUROPEEN

(12)

(45) Date de publication du fascicule du brevet :
06.03.91 Bulletin 91/10

(51) Int. Cl.⁵ : **A61K 33/04,** A61K 33/40, A61K 7/28

(21) Numéro de dépôt : **87904057.4**

(22) Date de dépôt : **19.06.87**

(86) Numéro de dépôt international :
**PCT/FR87/00232**

(87) Numéro de publication internationale :
**WO 87/07838 30.12.87 Gazette 87/29**

(54) **PROCEDE POUR CONDITIONNER UNE COMPOSITION ANTI-BACTERIENNE ET COMPOSITION ANTI-BACTERIENNE CONDITIONNEE.**

(30) Priorité : 23.06.86 FR 8609166

(43) Date de publication de la demande :
15.06.88 Bulletin 88/24

(45) Mention de la délivrance du brevet :
06.03.91 Bulletin 91/10

(84) Etats contractants désignés :
**AT BE CH DE FR GB IT LI LU NL SE**

(56) Documents cités :
**EP-A- 0 133 736**
**DE-A- 2 709 390**
**GB-A- 2 162 063**
**US-A- 4 473 550**

(73) Titulaire : **SOCIETE BIO SERAE S. A.**
**2, rue des Tendes**
**F-12400 Saint Affrique (FR)**

(72) Inventeur : **DEGRE, François**
**2, rue des Tendes**
**F-12400 Saint Affrique (FR)**

(74) Mandataire : **Barre, Philippe**
**Cabinet Barre-Gatti-Laforgue 95 rue des**
**Amidonniers**
**F-31069 Toulouse Cédex (FR)**

## Description

La présente invention concerne un procédé pour conditionner une composition anti-bactérienne, en vue d'empêcher sa dégradation pendant la période de stockage située entre la fabrication et la consommation. Elle concerne également une composition anti-bactérienne conditionnée selon le procédé.

La composition anti-bactérienne à conditionner selon le procédé faisant partie de la présente invention, contient de la lactoperoxydase, du thiocyanate, un donneur d'oxygène natif et peut être de la lactoferrine.

On sait que la présence du thiocyanate, de l'oxygène natif et de la lactoperoxydase dans un milieu aqueux produit un inhibiteur bactérien sous une forme d'ion du hypothiocyanate monovalent (OSCN) en équilibre acide-base avec sa forme acide (HOSCN). (voir par exemple EP 0 133 736).

La présence de l'eau est donc nécessaire pour faire démarrer la réaction ci-avant citée. Par conséquent le stockage de ces produits mélangés peut causer des dégradations. Le degré de la dégradation est fonction de la durée de stockage, et aussi de l'humidité du milieu.

Dans le brevet EP 0 133 736, on essaie de surmonter cette difficulté, soit par l'usage d'un milieu non-aqueux par exemple la glycerine ou le glycol de propylène, soit par limitation de la quantité d'eau dans la composition (10% maximum par poids). Le même brevet enseigne également l'usage du silica gel dans la composition à un taux de 1 à 5% par poids, ceci pour capter l'eau libre dans le milieu.

Dans le GB 2162063, on essaie de surmonter cette difficulté (sans le citer explicitement) en dispersant les matières actives dans une grande masse de poudre sèche. Ceci aide à minimiser la chance de rencontre entre les particules actives, d'abord pour dégager,en supposant que le degré d'humidité n'est pas nul, le peroxyde d'hydrogène et puis pour produire les ions (OSCN) en équilibre avec leur forme acide (HOSCN).

Une fois que le milieu est rendu aqueux, la mobilité de ces particules augmente en raison de la grande quantité d'eau qui facilite et/ou participe, en même temps, à la réaction. L'utilisation d'une grande masse de poudre sèche pour véhiculer les matières actives, rend l'usage d'une telle composition difficile notamment chez l'homme.

Dans tous les cas, soit pour les compositions décrites par EPO 133 736, soit pour celles décrites par GB 2 162 063, le risque de dégradation des matières actives est présent.

On connaît également de brevet FR 2 345 940 suivant lequel le donneur d'oxygène natif est couvert par une couche protectrice dont la matière devient perméable et/ou se dissout dans le milieu basique des intestins. Un exemple de telle matière est l'acétophtalate de cellulose. Le but de telle protection spécifique est la protection contre l'acidité gastrique. La composition anti-bactérienne revendiquée par ce brevet, vise uniquement les intestins dont le milieu est basique. Cette composition ne peut pas être utilisée pour traiter d'autres endroits dont le milieu est neutre ou acide comme par exemple la bouche, les yeux, la peau et le vagin.

Un but du procédé faisant partie de la présente invention est de conditionner une composition anti-bactérienne contenant le système " lactoperoxydase-thiocyanate-donneur d'oxygène natif" de telle sorte que la composition obtenue soit valable pour traiter les intestins comme les autres endroits ci-dessus cités.

Le donneur d'oxygène natif dans cette composition anti-bactérienne, peut être un système enzymatique oxydant ou un peroxyde soit organique soit inorganique.

Un autre but du dit procédé est de donner la possibilité de stocker une telle composition, à toutes concentrations voulues pour de longues périodes. Ceci tout en préservant l'intégralité de l'activité enzymatique à la dite composition.

Selon l'actuel procédé le peroxyde où l'un des composants du système enzymatique oxydant est isolé matériellement du reste de la dite composition.

A cet effet,suivant une disposition de l'invention, le procédé en faisant partie se caractérise essentiellement en ce que la matière utilisée pour effectuer cet isolement est soit hydrosoluble soit perméable dans les milieux, aqueux.

La présente invention, sera mieux comprise à la lecture de la description détaillée ci-dessous accompagnée par des exemples donnés à titre indicatif.

Les propriétés anti-bactériennes de la lactoferrine et du système (lactoperoxydase-thiocyanate-$H_2O_2$) sont bien connues. La lactoférrine capte le fer disponible dans le milieu et empêche le développement bactérien pour lequel ce fer est nécessaire. La lactoperoxydase joue un rôle de catalyseur pour aider la réaction entre le thiocyanate et le donneur d'oxygène pour produire les ions d'hypothiocyanate monovalent (OSCN) qui sont des inhibiteurs antibactériens. Ces ions existent en équilibre acide-base avec leur forme acide (HOSCN). Un milieu aqueux est nécessaire pour que cette réaction ait lieu. Dans cette description, le péroxyde ou le système enzymatique oxydant sont appelés donneurs d'oxygène natif. En effet le rôle de ces donneurs d'oxygène natif est de produire, dans le milieu aqueux nécessaire, des molécules de peroxyde d'hydrogène ($H_2O_2$). ces derniers oxydent, à l'aide de la lactoperoxydase, les ions de thiocyanate (SCN) pour produire les ions d'hypothiocyanate (OSCN).

Pour que la production de l'eau oxygénée (H$_2$O$_2$) et des ions d'hypothiocyanate soit assurée, un milieu aqueux est nécessaire. Ces deux étapes de réaction peuvent démarrer à un taux d'humidité supérieur à 2%, ceci à une vitesse relativement lente. Cette vitesse dépend de la concentration de molécules participant aux réactions dans la masse globale dans laquelle ces dernières existent. Il est évident que si la concentration de ces molécules est relativement élevée, à une humidité permettant la réaction, la vitesse de la réaction sera plus élevée. D'autre part, à des concentrations élevées de molécules actives et à un taux d'humidité inférieur à celui permettant les réactions ci-dessus citées, l'existence d'un oxydant fort en contact avec les enzymes et les protéines peut détériorer ces dernières.

Le procédé faisant partie de la présente invention, se base sur la séparation physique par une matière hydrosoluble ou perméable dans les milieux aqueux entre les enzymes et les protéines et tous autres composants capables de produire l'oxygène natif. Le terme " tous autres composants" signifie ici un ensemble capable de produire de l'oxygène natif d'une façon autonome.

De ce fait des concentrations plus élevées d'enzymes peuvent être utilisées, tout en évitant le risque, soit de faire démarrer des réactions produisant un effet antibactérien, soit de détèriorer les enzymes elles-mêmes.

Le donneur d'oxygène natif est nécessaire, pour les réactions ci-avant citées, dont les produits ont des effets antibactériens peut être, comme c'est connu, soit du peroxyde organique ou inorganique, soit un système enzymatique oxydant.

Le peroxyde inorganique peut être par exemple le péroxyde de mag nésium ou de sodium et le peroxyde organique peut-être par exemple le peroxyde de benzoyle.

Le système enzymatique oxydant peut être par exemple glucose-glucose oxydase ou autres (voire par exemple EP 0 133 736). Tous ces donneurs d'oxygène sont décrits dans la littérature.

Selon le procédé faisant partie de la présente invention, dans le cas d'utilisation d'un système enzymatique oxydant, un de ses composants est isolé du reste de la composition antibactérienne ; ce composant à isoler peut-être le glucose pour le système glucose, glucose-oxydase, et dans le cas d'utilisation d'un peroxyde soit organique, soit inorganique, ce peroxyde est isolé du reste de la composition anti-bactérienne.

La dite séparation selon l'invention peut être effectuée :

– soit par l'enrobage des granules du peroxyde ou de ceux de l'un des composants du système enzymatique oxydant, et de préférence le substrat, au moyen d'une couche protectrice hydrosoluble comme par exemple le saccharose, l'amidon, la gélatine, le carboxyméthyle cellulose ou équivalent.

– soit par des grains de gel sec, comme par exemple la pectine associée aux ions de calcium, dans lequel le peroxyde ou l'un des éléments du système enzymatique oxydant est contenu, la taille de maille de réseau du dit gel, une fois humidifié, devenant suffisamment grande pour permettre au moins le passage libre du peroxyde ou de l'un des deux composants du système enzymatique oxydant constitué d'un substrat et d'une enzyme.

Il est encore possible d'inclure dans la composition anti-bactérienne une enzyme capable d'hydrolyser le dit gel dans le milieu aqueux, ceci permet de détruire le dit réseau et libérer totalement la matière inclue à l'intérieur. Il est bien évident que la pectine peut être utilisée de même que n'importe qu'elle matière équivalente. Dans le cas d'utilisation de la pectine, la pectinase est à utiliser comme enzyme conduisant l'hydrolyse.

Pour réaliser ces granules de pectine renferment soit le peroxyde soit l'un des composants du système enzymatique oxydant, on dissout la pectine et la substance à renfermer dans une solution aqueuse, on fait tomber en pluie fine des gouttes calibrées dans une solution contenant des ions de calcium, les gouttes tombant forment des perles de gel de la pectine avec les ions de calcium, celles-ci renferment la dite substance. Les dites perles sont recueillies, séchées puis mélangées avec les autres composants pour former ainsi la composition antibactérienne.

Il est à noter que dans le cas d'utilisation d'un système enzymatique oxydant, comme par exemple le système glucose, glucose-oxydase, le substrat, c'est à dire le glucose dans notre cas , peut être soit fourni sous sa forme exploitable par l'enzyme "glucose-oxydase", soit fourni en utilisant d'autres produits capables de le produire lors de l'usage.

A titre d'exemple, on peut utiliser, à la place du glucose, l'amidon ou la malto dextrine, ceci, bien évidemment, en leur y associant les enzymes adequates lors de l'usage pour produire le glucose selon la réaction :

Amidon-alpha-amylase-Malto Dextrine-amyloglucosinase- Glucose

Dans ce cas là, l'amidon ou la malto dextrine est à isoler du reste de la composition anti-bacterienne qui contient, entre autres, soit l'alpha-amylase, soit l'amyloglucosinase seulement. De même on peut utiliser, à la place du glucose, le système fructose, glucose-isomerase, lactose-betagalactosidase.

Pour augmenter l'activité de la lactoferrine, autrement dit pour donner à la lactoferrine une très grande capacité de former des complexes avec le fer disponible dans le milieu, on incorpore dans la dite composition antibacterienne du bicarbonate d'un métal alcalin, comme par exemple le bicarbonate de sodium. Pour que la lactoferrine ait une bonne activité complexante, elle doit se trouver lors de son action, dans un milieu dont le bicarbonate représente environ de 0,1 à 1% par poids. Cette concentration du bicarbonate est de préférence de 0,6% par poids du milieu.

D'une façon générale la quantité d'oxygène à offrir pour oxyder le thiocyanate doit être le double de celle nécessaire pour cette oxydation. Par exemple si on incorpore xM de thiocyanate dans la composition, il faut incorporer une quantité suffisante du peroxyde ou de système enzymatique oxydant, pour produire 2xM de $H_2O_2$.

Ceci nous parait préférable pour s'assurer d'une utilisation complète de la quantité de thiocyanate.

Il est possible aussi d'utiliser de la lactoferrine exempte de fer, cette lactoférrine est connue sous le nom "apo-lactoferrine"(marque déposée).

Il est à noter aussi qu'il faut éviter les substances faisant concurrence avec la lactoferrine pour produire des complexes avec le fer disponible dans le milieu, ceci si ces substances malgré leur effet complexant, sont suceptibles de libérer tout ou partie de ce fer le rendant à nouveau disponible pour les bactéries. Par exemple ces substances peuvent être le tartrate, l'oxalate, et le citrate.

La composition antibactérienne conditionnée selon le procédé, peut être sous une forme de poudre sèche composée de granulés dont certains contiennent le peroxyde ou un des composants du système enzymatique enrobés dans une couche hydrosoluble. Cette couche peut être composée de saccharose, d'amidon, de carboxymethyle de cellulose ou équivalent.

Il est à noter que la technique d'enrobage est connue en elle-même dans l'industrie pharmaceutique.

Les granulés à isoler peuvent être renfermés dans des grains de gel, comme celui cité ci-dessus ou d'autres glucides ou polymères hydrosolubles.

Dans le cas d'utilisation d'un système enzymatique oxydant constitué d'un susbstrat et d'une enzyme, il est préférable d'enrober le substrat. Par exemple pour le système enzymatique glucose,glucose-oxydase, on enrobe le glucose par la dite couche isolante.

Cette composition peut être présentée dans des formes habituelles comme par exemple, sachet, gélules ou comprimés. Une telle composition peut être administrée oralement sous 'la forme de gélule ou de comprimé, elle peut être utilisée également comme gélules ou comprimés vaginaux. Sous sa forme de comprimé, elle peut être utilisée comme cachets à sucer.

Elle peut être aussi utilisée comme soluté, après l'avoir fait se dissoudre dans l'eau juste avant l'usage.

Une fois dissoute, elle peut être utilisée : en administration orale, comme bain de bouche, comme gargarisme, comme lavage vaginal thérapeutique ou spermicide, pour des besoins dermatologiques, otologiques, cosmétiques ou ophtalmologiques. Ceci bien évidemment à des dosages adéquats à chacun de ces usages. Des exemples de ces dosages seront donnés plus tard.

Pour les usages externes, un agent épaississant à l'état sec comme par exemple le carboxyméthyle de cellulose et/ou un agent tensioactif courant peuvent être ajoutés à la dite poudre, ceci permet l'obtention d'une forme plus ou moins pâteuse, une fois que la dite composition est humidifiée.

Cette forme pâteuse peut être par exemple préférée pour les besoins dermatologiques.

La composition anti-bactérienne, conditionnée selon l'invention, peut être incorporée dans une pâte ferme comme par exemple la pâte à macher, cette pâte sera malaxée et humidifiée lors de l'usage.

La composition anti-bactérienne, conditionnée selon l'invention, peut être incorporée dans des aliments pour animaux.

Dans ce qui suit, nous donnons quelques exemples des doses de matières actives à inclure dans les différents produits pour obtenir ainsi, dans chaque cas une composition antibactérienne :

PATE A MACHER (par chaque tablette)

BAIN DE BOUCHE ( par 25 ml de solution)

GARGARISME (par 25 ml de solution)

| | |
|---|---|
| lactoférrine | de 5 à 50 mg |
| lactoperoxydase | de 0,1 à 1 mg |
| thiocyanate | 5 mg |
| glucose | de 25 à 50 mg |
| glucose oxydase | de 0,1 à 1 mg |

L'ensemble constitué par le glucose et le glucose-oxydase peut être remplacé par exemple, par le magnésium peroxyde à un taux de 20 à 100 mg et de préférence 35 mg.

COLLYRE(par 5 à 25 ml)

Les quantités de substances actives inclues dans le bain de bouche par 25 ml peuvent être inclues dans une quantité de soluté de 5 à 25 ml à usage ophtalmologique.

SOLUTE à usage dermatologique (par 100 ml de soluté)

| | | |
|---|---|---|
| lactoferrine | de | 10 à 100 mg |
| lactoperoxydase | de | 3 à 10 mg |
| thiocyanate | de | 25 à 35 mg |
| glucose | de | 25 à 100 mg |
| glucose oxydase | de | 0,1 à 2 mg |

L'ensemble constitué de glucose et de glucose oxydase peut être remplacé, par exemple, par le peroxyde de magnésium à un taux variant de 35 à 150 mg.

SOLUTE à usage cosmétique (par 100 ml de soluté)

| | |
|---|---|
| lactoférrine | 5 mg |
| lactoperoxydase | 3 mg |
| thiocyanate | 25 mg |
| $MgO_2$ | 35 mg |

## <u>COMPRIME GELULE POUR INFECTION INTESTINALE</u> (par dose)

| | | | |
|---|---|---|---|
| lactoferrine | de | 30 à 150 | mg |
| et de préférence | de | 60 à 100 | mg |
| lactoperoxydase | de | 4 à 10 | mg |
| et de préférence | de | 6 à 8 | mg |
| glucose | de | 100 à 500 | mg |
| et de préférence | de | 200 | mg |
| glucose oxydase | de | 0,5 à 2 | mg |
| et de préférence | de | 1 | mg |

L'ensemble constitué de glucose et de glucose oxydase peut être remplacé par exemple par le peroxyde de magnésium à un taux de 25 à 150 mg et de préférence de 35 mg.

## <u>COMPRIME OU GELLULE</u> à usage gynécologique antibactérien (par dose)

:

| | | | |
|---|---|---|---|
| lactoférrine | de | 5 à 60 | mg |
| lactoperoxydase | de | 1 à 10 | mg |
| et de préférence | de | 4 à 5 | mg |
| thiocyanate | de | 24 à 35 | mg |
| glucose | de | 100 à 200 | mg |
| glucose-oxydase | de | 1 à 2 | mg |

L'ensemble constitué de glucose et de glucose-oxydase peut être remplacé par le peroxyde de magnésium à un taux de 25 à 100 mg.

COMPRIME OU CELLULE à usage spermicide (par dose)

| lactoférrine | au choix |
|---|---|
| lactoperoxydase | 10 mg |
| thiocyanate | 25 mg |
| MgO$_2$ | 35 mg |

Il faut noter que la lactoférrine n'a pas d'effet spermicide connu, elle peut être incorporée dans la composition à titre d'agent d'hygiène en raison de son pouvoir anti-bactérien.

Il faut également noter que la pression osmotique dans les deux parties de l'ovule contenant la composition antibactérienne ou spermicide doit être égalisée. Ceci pour éviter la migration des particules actives d'un côté à l'autre de l'ovule. Si la pression osmotique ne peut pas être égalisée, la séparation de deux moitiés de l'ovule par un film hydrosoluble ou thermofusible diminue beaucoup cette migration.

## Revendications

1. Procédé pour conditionner une composition anti-bactérienne sous forme sèche contenant de la lactoperoxydase, du thiocyanate, un donneur d'oxygène natif et peut être de la lactoferrine en vue d'empêcher sa dégradation pendant la période située entre la fabrication et la consommation, le donneur d'oxygène natif pouvant être un système enzymatique oxydant ou un peroxyde soit organique soit inorganique, le peroxyde ou l'un des composants du système enzymatique oxydant étant isolé matériellement du reste de la dite composition, procédé étant caractérisé en ce que la matière utilisée pour effectuer cet isolement est soit hydrosoluble soit perméable dans les milieux aqueux.

2. Procédé selon la revendication 1 caractérisé en ce que le peroxyde ou l'un des composants du système enzymatique oxydant est isolé du reste de la dite composition :
   – soit par une couche protectrice hydrosoluble formant des micro capsules dont la matière peut être par exemple le saccharose, l'amidon, le carboxyméthyle de cellulose ou équivalent,
   – soit par des grains de gel sec comme par exemple la pectine associée aux ions de calcium dans lesquels le peroxyde ou l'un des composants du système enzymatique oxydant est contenue, la taille de maille de réseau du dit gel, une fois humidifiée, devenant suffisamment grande pour permettre, au moins, le passage libre du peroxyde ou de l'un des deux composants du système enzymatique oxydant constitué d'un substrat et d'une enzyme.

3. Procédé selon les revendications 1 et 2 caractérisé en ce qu'on incorpore dans la dite composition, dans le cas ou la lactoferrine en fait partie, du bicarbonate d'un métal alcalin comme par exemple le bicarbonate de soude, ceci à telle concentration que la lactoferrine se trouve, lors de son action dans un milieu contenant de 0,1% à 1% par poids et de préférence de 0,6% par poids dudit bicarbonate.

4. Composition antibactérienne sous forme de poudre sèche caractérisée en ce qu'elle est conditionnée selon l'une quelconque des revendications 1, 2 et 3 puis diluée dans un milieu aqueux à une concentration adéquate pour les usages suivants :
   – l'administration orale,
   – les besoins dermatologiques,
   – les besoins ophtalmologiques,
   – les besoins otologiques,
   – le lavage vaginal,
   – comme gargarisme,
   – comme bain de bouche.

5. Composition anti-bactérienne sous forme de comprimé caractérisée en ce que les substances actives sont conditionnées selon l'une quelconque des revendications de 1 à 3, les formes et les dosages étant adéquats aux usages suivants :

– l'administration orale,

– comprimé à usage gynécologique,

– cachet à sucer.

6. Composition antibactérienne sous forme de gélules caractérisée en ce que les substances actives sont conditionées selon l'une quelconque des revendications 1 à 3, les formes des dites gélules et leurs dosages étant conçu pour les usages suivants :

– l'administration orale,

– gélule à usage gynécologique.

7. Composition antibactérienne destinée à être utilisée comme pâte à macher caractérisée en ce qu'elle est constituée d'une pâte ferme contenant des substances actives conditionnée selon l'une quelconque des revendications 1 à 3.

8. Composition antibactérienne à mélanger aux aliments pour animaux, caractérisée en ce qu'elle est constituée d'une poudre conditionnée selon l'une quelconque des revendications de 1 à 3.

## Ansprüche

1. Verfahren zum Verpacken einer antibakteriellen Zubereitung in trockener Form, die Lactoperoxidase, Thiozyanat, einen natürlichen Sauerstoffspender und eventuell Lactoferrin im Hinblick darauf enthält, ihren Zerfall im Zeitraum zwischen ihrer Herstellung und ihrem Verbrauch zu verhindern, wobei der natürliche Sauerstoffspender ein oxidierendes Enzymsystem oder ein organisches bzw. anorganisches Peroxid sein kann, und das Peroxid oder eine der Komponenten des Enzymsystems von der übrigen Zubereitung materiell isoliert ist, dadurch gekennzeichnet, daß der zur Durchführung dieser Isolierung verwendete Stoff entweder wasserlöslich oder in wäßrigen Medien durchlässig ist.

2. Verfahren nach Anspruch 1, dadurch gekennzeichnet, daß das Peroxid oder eine der Komponenten des oxidierenden Enzymsystems

– entweder durch eine wasserlösliche Schutzschicht, die Mikrokapseln bildet, deren Material, beispielsweise Saccharose, Stärke, Carboxymethylzellulose oder dgl. sein kann,

– oder durch Trockengelkörner wie, beispielsweise, das mit den Calciumionen assoziierte Pektin, in denen das Peroxid oder eine der Komponenten des oxidierenden Enzymsystems enthalten ist, wobei die Maschenweite des Gels, sobald es befeuchtet ist, groß genug wird, um zumindest den freien Durchgang des Peroxids oder einer der beiden Komponenten des aus einem Substrat und einem Enzym gebildeten oxidierenden Enzymsystems zu ermöglichen, von der übrigen Zubereitung isoliert wird.

3. Verfahren nach den Ansprüchen 1 und 2, dadurch gekennzeichnet, daß in die vorerwähnte Zubereitung, falls sie Lactoferrin enthält, ein Bicarbonat eines alkalischen Metalls, wie zum Beispiel Sodiumbicarbonat, eingebracht wird, und dies in der Konzentration, in der sich die Lactoferrin bei ihrer Wirkung in einem Medium befindet, das 0,1 bis 1 Gewichts-%, vorzugsweise 0,6 Gewichts-%, des Bicarbonats enthält.

4. Antibakterielle Zubereitung in Trockenpulverform, dadurch gekennzeichnet, daß sie nach einem der Ansprüche 1, 2 und 3 verpackt wird, dann in einem wäßrigen Medium in einer für die folgenden Zwecke geeigneten Konzentration verdünnt wird :

– orale Verabreichung,

– dermatologische Erfordernisse,

– ophtalmologische Erfordernisse,

– ohrenheilkundliche Erfordernisse,

– Vaginalspülung,

– Gurgelmittel,

– Mundspülung.

5. Antibakterielle Zubereitung in Tablettenform, dadurch gekennzeichnet, daß die Wirkstoffe nach einem der Ansprüche 1 bis 3 verpackt werden, wobei sich die Formen und die Dosierungen für die folgenden Zwecke eignen :

– orale Verabreichung,

– Tablette für gynäkologische Zwecke,

– Oblatenkapsel zum Lutschen.

6. Antibakterielle Zubereitung in der Form von Gelatinekapseln, dadurch gekennzeichnet, daß die Wirkstoffe nach einem der Ansprüche 1 bis 3 verpackt werden, wobei die Formen dieser Kapseln und ihre Dosierungen für die nachstehenden Zwecke ausgebildet sind :

– orale Verabreichung,

– Gelatinekapsel für gynäkologische Zwecke.

7. Antibakterielle Zubereitung, die für die Verwendung als Kaumasse bestimmt ist, dadurch gekennzeichnet, daß sie aus einer festen, nach einem der Ansprüche 1 bis 3 verpackte Wirkstoffe enthaltenden Masse besteht.

8. Antibakterielle Zubereitung zum Einmischen in Tiernahrungsmittel, dadurch gekennzeichnet, daß sie aus einer nach einem der Ansprüche 1 bis 3 verpackten Pulver besteht.


**Claims**

1. Method for conditioning an anti-bacterial compound in dry form containing lactoperoxydase, thiocyanate, a natural oxygen donor and, possibly, lactoferrin with a view to preventing its degradation during the period between its preparation and its consumption, in which the native oxygen donor can be an oxidising enzymatic system or a peroxide, either organic or anorganic, with the peroxide or one of the constituents of the oxidising enzymatic system being isolated materially from the rest of the compound, characterised in that the material used to bring about this isolation is either water-soluble, or permeable in aqueous medias.

2. Method according to claim 1, characterised in that the peroxide or one of the constituents of the oxidising enzymatic system is isolated from the rest of the compound :
    – either through a protective water-soluble layer forming microcapsules, the material of which may be, for example, saccharose, starch, carboxymethylcellulose or equivalent,
    – or through granules of dry gel as for example the pectin associated with the calcium ions in which the peroxide or one of the constituents of the oxidising enzymatic system is contained, with the net mesh size of the said gel, once it has been moistened, becoming large enough to allow, at least, for free passage of the peroxide or of one of the constituents of the oxidising enzymatic system consisting of one substrate and one enzyme.

3. Method according to claims 1 and 2, characterised in that bicarbonate of an alcaline metal, as for example sodium bicarbonate, is added to said compound when lactoferrin is a part of it, in a concentration such that the lactoferrin finds itself, during its action, in a medium containing 0,1 to 1% by weight, preferably 0,6% by weight of said bicarbonate.

4. Anti-bacterial compound in dry powder form, characterised in that it is conditioned according to any one of the claims 1, 2 and 3, then diluted in an aqueous medium up to a concentration that is suitable for the following purposes :
    – oral administration,
    – dermatological purposes,
    – ophtalmological purposes,
    – otological purposes,
    – vaginal cleansing,
    – gargle,
    – mouth cleanser.

5. Anti-bacterial compound in the form of a tablet, characterised in that the active substances are conditioned according to one of the claims 1 to 3, in the forms and dosages suitable for the following purposes:
    – oral administration,
    – tablet for gynaecological purposes,
    – wafer for sucking.

6. Anti-bacterial compound in the form of capsules, characterised in that the active substances are conditioned according to any one of the claims 1 to 3, with the forms of said capsules and their dosages being suitable for the following purposes :
    – oral administration,
    – capsule for gynaecological purposes.

7. Anti-bacterial compound designed for being used as a chewing gum, characterised in that it consists of a solid gum containing active substances conditioned according to any one of the claims 1 to 3.

8. Anti-bacterial compound for mixing into animal feed, characterised in that it consists of a powder conditioned according to any one of the claims 1 to 3.